# EUROPEAN PATENT APPLICATION

(11) **EP 2 474 552 A1**
(43) Date of publication of application: **11.07.2012**
(21) Application number: 11000103.9
(22) Date of filing: 07.01.2011
(51) Int. Cl.: C07H 15/26, A61K 31/7048, A61P 35/04

(54) **New saccharide mimics and their use as inhibitors of angiogenesis and metastasis formation**

(71) Applicant: Deutsches Krebsforschungszentrum, 69120 Heidelberg (DE)
(72) Inventor: Schwartz-Albiez, Reinhard, 69251 Gaiberg (DE); Marano, Grazia, 67317 Altleiningen (DE); Frei, Eva, 69120 Heidelberg (DE); Frank, Martin, 74523 Schwäbisch Hall (DE); Meister, Betina, 80336 München (DE)
(74) Representative: Schüssler, Andrea

(57) **Abstract**

The present invention relates to saccharide mimics which show a better biological activity at low concentrations as known saccharide mimics in inhibiting angiogenesis and inhibiting formation of metastasis by inhibiting adhesion and/or migration of human cancer cells, particularly in concentrations lower than 5 mM, and to pharmaceutical compositions comprising these saccharide mimics. The compounds of the present invention are useful as mimics of cell surface structures and, thus, for influencing cell-cell and cell-extracellular (ECM) interactions, which are controlled by bonds between protein-protein, saccharide-saccharide or saccharide-lectin on the cell-surface.
Representative compounds of the present invention are for example:

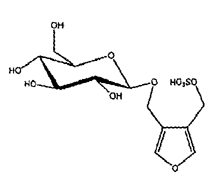

## Description

### BACKGROUND OF THE INVENTION

The present invention relates to saccharide mimics, which are particularly useful as inhibitors of angiogenesis and metastasis formation.

The vast majority of malignant or metastasizing tumor diseases are not yet curable. With only a few exceptions, there are no curative forms of therapies, such that novel treatment methods are urgently needed. For example, the inhibition of the tumor-associated angiogenesis, i.e. directed against the formation of new blood vessels, is a new option for treating solid tumors and haematological malignancies (Moehler, M. et al., Curr. Pharm. Des., 2004: 11, 1221-1234; Ribatti, D. et al., Stem Cell Development, 2004: 13, 484-495). An effective agent against tumor angiogenesis should fulfil multiple criteria, such as: a) efficiency against different types of tumors, b) small risk of resistance, c) efficiency against different mechanisms of tumor-associated angiogenesis, d) low toxicity, and e) possible combination with other forms of therapies, e.g. chemotherapy. However, most of the already existing active agents do not meet these criteria.

Up to now, the previously used or tested active agents for the inhibition of the tumor-associated angiogenesis are predominantly based on the protein portion of specific angiogenic factors or receptors. For example, since the vascular endothelial growth factor (VEGF) stimulates the growth of new blood vessels, anti-VEGF therapies are important in the treatment of certain tumors. These treatments involve monoclonal antibodies such as bevacizumab (tradename "Avastin"), which show therapeutic efficiency in mouse tumor models and in an increasing number of human tumors. Furthermore, drugs against the corresponding VEGF-receptor, e.g. semaxanib (SU5416), vandetanib (ZD6474) or CP-547,632 (Malik, A. K. and H.-P. Gerber, Targets, 2003: 2, 48-57) and drugs with a wider spectrum, e.g. thalidomide, interferon, such as IFN-α or IFN-β, cele-coxib, ZD6126 and fumagillin are in use or rather currently part of clinical studies as anti-angiogenic agents. Further drugs, such as lanildomide (tradename "Revlimid"), a derivative of thalidomide, induce tumor cell apoptosis directly and indirectly, e.g. by anti-angiogenic effects.

Tumor angiogenesis is the proliferation of a network of blood vessels that penetrates into cancerous tissue, supplying nutrients and oxygen to the tumor and removing waste products from the tumor. Tumor angiogenesis actually starts with cancerous tumor cells releasing molecules that send signals to surrounding normal host tissue. This signaling activates certain genes in the host tissue that, in turn, make proteins to encourage growth of new blood vessels.

More specifically, tumor angiogenesis comprises several overlapping phases where the endothelial cells (EC) are involved: (1) activation of the endothelial cells, i.e. biological signals known as angiogenic growth factors activate receptors on endothelial cells present in pre-existing blood vessels; (2) adhesion and migration of the endothelial cells and formation of a provisional blood vessel, i.e. the activated endothelial cells begin to release enzymes called proteases that degrade the basement membrane to allow endothelial cells to escape from the original (parent) vessel walls. The endothelial cells then proliferate into the surrounding matrix and form solid sprouts connecting neighbouring vessels. As sprouts extend towards the source of the angiogenic stimulus, endothelial cells migrate in tandem, using adhesion molecules, called integrins; (3) stabilisation of the new blood vessel by perivascular cells, e.g. fibroblasts and pericytes, i.e. the sprouts then form loops to become a full-fledged vessel lumen as cells migrate to the site of angiogenesis. Sprouting occurs at a rate of several millimetres per day and enables new vessels to grow across gaps in the vasculature. The tumor vessels show several differences to normal blood vessels and capillaries, i.e. incomplete and irregularly formed basal membranes and large interendothelial gaps.

Anti-angiogenic drugs which are intended for treating solid tumors as well as haematological malignancies lead to a certain improvement of the progression-free phase and the median overall survival of the patients, also in combination with chemotherapy. However, these drugs cannot prevent the overall progression of the malignant diseases and do not have a curative effect. This is mostly due to the fact that tumor cells can develop a certain resistance against this anti-angiogenic treatment, e.g. by using mechanisms of vascularization other than VEGF-induced angiogenesis. Furthermore, for some (unknown) reason some patients remain irresponsive to this therapy. Moreover, some therapeutics are associated with severe side effects, e.g. disorders of the peripheral or dycardia) und heart failure.

Thus, the development of further angiogenesis inhibitors with an independent mechanism of action is necessary. A new generation of angiogenesis inhibitors are based on the fact that activated endothelial cells adhere to the extracellular matrix, migrate and form cell-cell contacts during the initiation of tumor associated angiogenesis. Therefore, cell-cell and cell-extracellular matrix (ECM) interactions, which are controlled by bonds between protein-protein, saccharide-saccharide or saccharide-lectin on the cell surface, are necessary. Novel angiogenesis inhibitors represent mimics of these specific cell surface structures, e.g. saccharide mimics. On the one hand, such saccharide mimics inhibit central functions of the activation of endothelial cells and, on the other hand, significantly influence the interaction between hematopoietic cells and endothelial cells. Thus, saccharide mimics prevent the formation of new blood vessels which are necessary for tumor growth and they induce an anti-inflammatory effect. Furthermore, they inhibit the interaction of metastasizing tumor cells with cells of the target organ. Thus, saccharide mimics have an enormous potential, since they are not only applicable for inhibiting angiogenesis but also for inhibiting metastasis.

Naturally occurring saccharides are very complex and branched molecules, whereas their binding molecules are highly specific for the three-dimensional structure of the saccharide ligands, a reason why their synthesis is very complicated and expensive. Thus, saccharide-mimics, such as vascular-inhibiting glycan analogs (VIGA) with a fixed stereochemistry of the core molecule have been synthesized, since their synthesis is easier and cheaper. Previous studies have already shown that such saccharide mimics containing furan as the core structure, derivatized with galactose, inhibit not only the cell adhesion of melanoma cells to the ECM-protein fibronectin but also migration/invasion of the melanoma cells through the matrix (Kim, E. Y.-L., Synthese von multivalenten Saccharidmimetika als Liganden für Zellmembran-Lectine und Inhibitoren der Zelladhäsion und Invasion, Heidelberg, Univ., Diss., 2002; Kim, E. Y.-L., ChemBioChem, 2005: 6, 422-431). However, a significant disadvantage of the already present saccharide mimics is that high concentrations are necessary for biological activity, e.g. 20-40 mM VIGA or 5 mM VIGA (Gronewold, C., In vitro Untersuchungen zur Wirkung von Saccharidmimetika auf verschiedene Schritte der Metastasierung von malignen Melanomzellen, Heidelberg, Univ., Diss., 2005), which limits their tolerability for patients.

Thus, the problem of the present invention is the provision of further saccharide mimics, which show a better biological activity than known saccharide mimics in inhibiting angiogenesis and formation of metastasis by inhibiting the adhesion and/or the migration of human cancer cells at low concentrations, particularly lower than 5 mM.

The problem is solved by the subject-matter of the present claims 1, 3, 5, 7 and 9. Preferred embodiments are the subject-matter of the dependent claims.

### SUMMARY OF THE INVENTION

The present invention relates to saccharide mimics which show a better biological activity at low concentrations as known saccharide mimics in inhibiting angiogenesis and inhibiting formation of metastasis by inhibiting adhesion and/or migration of human cancer cells, particularly in concentrations lower than 5 mM, and to pharmaceutical compositions comprising these saccharide mimics. The compounds of the present invention are useful as mimics of cell surface structures and, thus, for influencing cell-cell and cell-extracellular (ECM) interactions, which are controlled by bonds between protein-protein, saccharide-saccharide or saccharide-lectin on the cell-surface.

### DETAILED DESCRIPTION OF THE INVENTION

The present invention relates to saccharide mimics showing a better biological activity as known saccharide mimics in inhibiting angiogenesis and formation of metastasis by inhibiting adhesion and/or migration of human cancer cells at low concentrations, particularly lower than 5 mM. Thus, the present invention relates to saccharide mimics, which have been optimized by modifying the structure of known saccharide mimics and to pharmaceutical compositions comprising these saccharide mimics.

The starting structure of the present invention for the optimization the biological activity is a molecule with furan as the core structure, which is derivatized with galactose, i.e. 3-β-D-galactopyranosyl-oxymethyl-4-sulfato-methyl-furan (GSF).

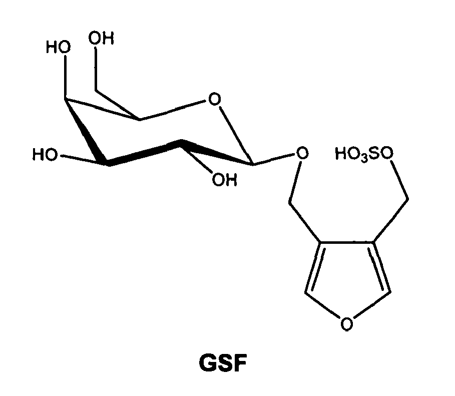

In previous studies, it has been shown that GSF completely inhibits the adhesion of specific murine and human melanoma cell lines, i.e. murine B16F10 cells and human WM-115 cells, to the ECM proteins fibronectin at 40 mM and fibrinogen at 20 mM or rather 10 mM. GSF also decreased the activation of the matrix-metalloproteinase-2 (MMP-2) and inhibited the migration of WM-115 and WM-266-4 cells at 5 mM (Meister, Bettina: Synthesis of new saccharide model substances to study sialic acid recognition, PhD Thesis, University of Heidelberg, Fakultät für Biowissenschaften, 2004). Furthermore, the inhibition of cell migration into a wound has been investigated with two-dimensional migration assays with a defined wounding of an intact cell-monolayer of WM-115 and WM-266-4 cells. GSF inhibited this process in a concentration dependent manner up to 5 mM for WM-115 and 2.5 mM for WM-266-4, where no detectable migration was observed (Gronewold, C., Proc. Amer. Assoc. Cancer Res., 2004: 45, Abstr. #1024; Gronewold, C., In vitro Untersuchungen zur Wirkung von Saccharidmimetika auf verschiedene Schritte der Metastasierung von malignen Melanomzellen, Heidelberg, Univ., Diss., 2005). A significant disadvantage of the saccharide mimic GSF, however, is that high concentrations are necessary in order to achieve the inhibitory effect, i.e. the use of saccharide mimics in millimolar concentrations is only practicable in vitro, without any cytotoxic effects. However, there is a need for an improvement of the biological activity for in vivo studies and treatments. Therefore, a method for optimization of the lead-structure GSF has been developed, so that a better effect on angiogenesis and metastasis inhibition occur at concentrations of about 5 mM and lower.

According to the present invention, the optimization of the lead-structure GSF is done either via a) the substitution of the saccharide-portion (galactose) with other mono- or disaccharides, b) the substitution of the sulfate group with other functional group(s) and/ or c) the substitution of the core structure furan with other heterocyclic systems by means of molecular modelling and specific synthesis, respectively, according to Kim, E. Y.-L. et al., CheniBioChem, 2005: 6, 422-431.

Thus, the present invention concerns compounds that are represented by the general Formula (I): 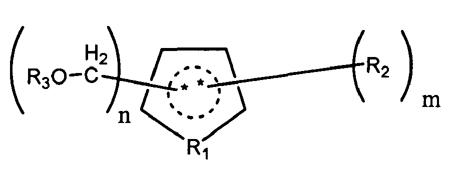 wherein
- n, m =: 1, 2, 3 with n + m ≤ 4
- R₁ =: N-H or O
- R₂ =: Alkyl-R₄
- R₃ =: saccharide selected from the group consisting of glucose, galactose, and the corresponding uronic acids, optionally having at least one of the saccharide-OH groups substituted by a R₄ residue and/or having at least one of the saccharide-OH groups oxidized to O
- R₄ =: H, halogen, -NH-C(O)-R₅, -N(R₆)₂, NR₆, NO₂, -O-S(= O)₂-O-R₆, S(= O)₂-O-R₆, -O-P(= O)(-O-R₆)₂, -OR₆, -O-C(O)-R₅, -S-R₆
- R₅ =: linear C₁ to C₄ alkyl
- R₆ =: H, linear C₁ to C₄ alkyl
with the exception of the compound 3-(β-D-galactopyranosyl-oxymethyl)-4-(sulfato-methyl)-furan (GSF), i.e. a compound of Formula (I) where R₁ = O, R₂ = CH₂-O-S (= O)₂ -O-H, R₃ = unsubstituted galactose, m = 1, n = 1.

The saccharide can be in any conformation, i.e. in the D- or L-form and in the α- or β-form, respectively.

The OH-groups of the saccharide can be unmodified, singly or multiply substituted or modified. Optionally at least one of the saccharide-OH groups is substituted by a R₄ residue and/or having at least one of the saccharide-OH groups oxidized to O.

If not stated otherwise, in the present invention the "alkyl" residue (preferably: C₁ to C₆) can be linear, branched or cyclic, unsubstituted or substituted. Preferred alkyl residues are methyl, ethyl, n-propyl, iso-propyl, n-butyl, tert-butyl, n-pentanyl, n-hexanyl. The term "alkyl", also in word combinations such as alkylsulfonyl, alkylamino or alkoxycarbonyl etc., mean both unbranched and branched possible compounds. The same also applies to the corresponding cyclic compounds.

The core molecule can be any heterocyclic saturated or unsaturated 5-member ring having O or N as heteroatom, i.e. pyrrol, furan, pyrrolidine or tetrahydrofuran. The core molecule can be substituted in addition to the "R₂"and "CH₂OR₃" substituents as mentioned in Formula (I) simply or multiply with any suitable functional group, such as hydroxy-, amino-, nitro-, carboxy-, carboxamido-, keto-, sulfoxide-, sulfon-, sulfonic acid, sulfate-, phosphoric acid, phosphonic acid, mono/di/trialkylamide-, halogens, thioether, thioester or thiols.

Within the meaning of the invention, all residues are considered combinable unless stated otherwise in the definition of the residues. All conceivable subgroupings thereof are considered to be disclosed.

Preferred compounds of the present invention are e.g. 3-(β-D-galactopyranosyloxymethyl)-4-(sulfonatomethyl)-furan (called "D-sulfonic acid") or 3-(β-D-glucopyranosyl-oxymethyl)-4-(sulfatomethyl)-furan (called "GluSF") having the chemical structures: 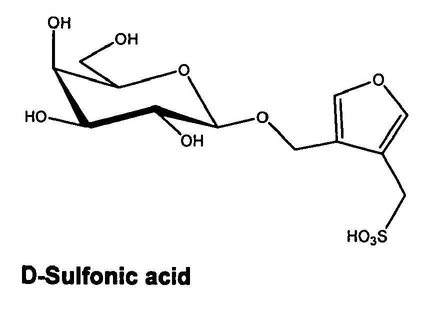 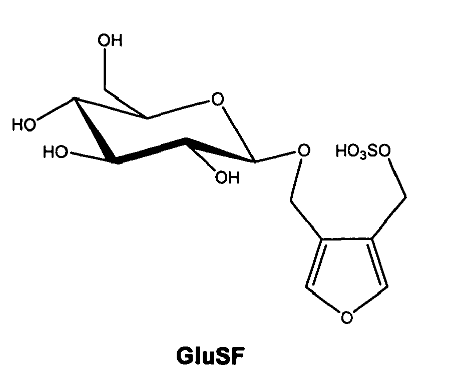 and 3-(β-D-galactopyranosyl-oxymethyl)-4-(fluoro-methyl)-furan (called "GFF") having the chemical structure:

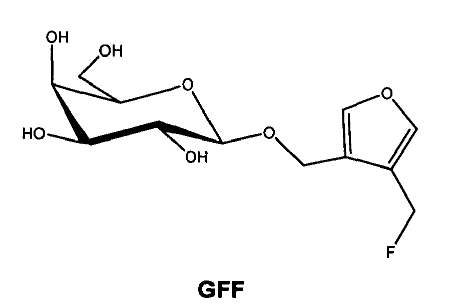

Particularly preferred is 3-(6-sulfato-β-D-galactopyranosyl-oxymethyl)-4-(sulfatomethyl)-furan (called "GSSF") having the chemical structure:

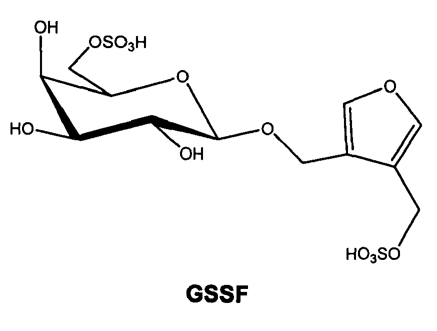

For the synthesis, derivatives of the saccharides comprising protecting groups or functionalized saccharides are used. The protecting groups can comprise benzoyl, silyl-, dimethoxytrityl- or acetyl-groups. The functionalized saccharides are saccharides, wherein the hydroxyl group is substituted with amino-, nitro-, carboxy-, carboxamido-, keto-, sulfoxide-, sulfon-, sulfonic, sulphuric acid and its alkylesters, sulfate-, phosphoric acid, phosphonic acid, mono/bis/trialkylamides, halogens, thioether, thioester or thiols.

The synthesis of the compounds of the present invention is described in detail in the example section.

In one aspect of the present invention, GSF is modified by substituting galactose with L-glucose or D-glucose, wherein the substitution with D-glucose is more preferred. This substitution leads e.g. to the saccharide mimic 3-(β-D-glucopyranosyl-oxymethyl)-4-(sulfatomethyl)-furan ("GluSF").

In another aspect, the present invention provides an optimized GSF by substituting the negatively charged sulfate-group with a halogen. In a preferred embodiment, the negatively charged sulfate-group of GSF is substituted with fluorine. This substitution leads e.g. to the saccharide mimic 3-(β-D-galactopyranosyl-oxymethyl)-4-(fluoro-methyl)-furan ("GFF").

In still another aspect, the present invention provides an optimized GSF by including a further sulfate-group. In a preferred embodiment, a second sulfate-group is included at the C-6-position of galactose. This modification leads e.g. to the saccharide mimic 3-(6-sulfato-β-D-galactopyranosyl-oxymethyl)-4-sulfatomethyl-furan ("GSSF").

The compounds of the present invention were analyzed by means of a migration-assay, called "wound healing assay", that determines the inhibition-rate of the compounds on human melanoma cell line migration. Another migration assay, the so-called the Oris^{™} cell migration assay, is also applicable to examine the cell migration inhibitory effects of the compounds of the present invention. The used melanoma cells lines were WM-115 and WM-266-4, which are accepted in-vitro model cell systems. The human cell line WM-115 is derived from the primary tumor of a vertical growth phase (VGP) melanoma, whereas the cell line WM-266-4 is derived from the corresponding cutaneous metastasis. The "wound healing assay" is applicable to examine the cell migration inhibitory effects of the compounds of the present invention, which is described in further detail in the Examples. Furthermore, the "wound healing assay" is based on the fact that when wounded or scratched, cell monolayers respond to the disruption of cell-cell contacts and an increased concentration of growth factors at the wound margin by healing the wound through a combination of proliferation and migration, according to Yarrow, J.C., et al., BMC Biotechnol., 2004: 4, 21-29. In order to investigate the cell migration an intact cell monolayer is wounded with a pipette tip.

If one of the synthesized chemical compounds shows an inhibitory effect on the migration of human melanoma cell lines at concentrations of about 5 mM and lower, this chemical compound is further checked for its biological efficiency by means of an adhesion assay. Suitable melanoma cells lines are WM-115 and WM-266-4. A suitable human endothelial cell line is HBMEC-60. The adhesion assay is described in further detail in the Examples. The adhesion-assay is a modified version of the method as described in Oliver, M. H. et al., J. Cell Science, 1989: 92, 513-518 and by Gronewold, C., In vitro Untersuchungen zur Wirkung von Saccharidmimetika auf verschiedene Schritte der Metastasierung von malignen Melanomzellen, Heidelberg, Univ., Diss., 2005.

While checking the biological efficiency of these synthesized chemical compounds, their toxicity is also determined by means of a sulforhodamin-B assay according to Gronewold, C., In vitro Untersuchungen zur Wirkung von Saccharidmimetika auf verschiedene Schritte der Metastasierung von malignen Melanomzellen, Heidelberg, Univ., Diss., 2005.

Furthermore, the biodegradability is additionally determined by means of computer-based methods, which are known in the art.

In addition, starting from the existing results the inventors have investigated the influence of some of the compounds of the present invention on the angiogenesis process according to known in-vitro models (e.g. "Matrigel-Assay", "Tubing-Assay")

The results of the different experiments confirm that :
1. The compounds of the present invention have an inhibitory influence on the endothelial cell mediated angiogenesis already at an early stage.
2. The compounds of the present invention do not have a negative influence on the vitality and proliferating ability of the endothelial cells
3. In view of the inhibitory effect on the adhesion also later phases of the angiogenesis will be inhibited. This means that the whole process of building new vessels will be inhibited. Thus, the compounds of the present invention are perfect anti-angiogenic compounds.

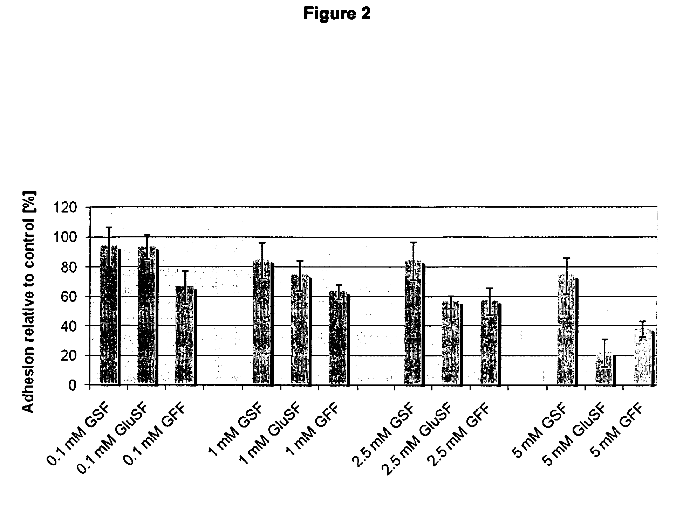

With regard to the adhesion of human melanoma cells to ECM, GluSF inhibits the adhesion of human melanoma cells to ECM particularly at concentrations of 0.1 - 5 mM, more particular at concentrations of 0.1, 1, 2.5 and 5 mM. For example, at a concentration of 0.1 mM GSF and GluSF are equally effective, wherein GluSF is much more effective at concentrations between 1 and 5 mM, more particular at concentrations of 1, 2.5 and 5 mM. At a concentration of 5 mM, GluSF inhibited the adhesion of WM-266-4 cells to fibronectin by 79%, while GSF showed an inhibition value of only 26% (see Fig. 2). Furthermore, GluSF is much more effective in inhibiting the migration of human melanoma cells compared to GSF, particularly at concentrations of 1 - 5 mM, more particular at concentrations of 1, 2.5 and 5 mM. For example, GluSF inhibits the migration of human melanoma cells already at a concentration of 1 mM, wherein GSF inhibits the migration of human melanoma cells only at concentrations greater than or equal to 2.5 mM (see Table 1). Thus, the compound GluSF represents an optimization of the compound GSF.

Furthermore, GFF is an optimized compound in comparison to the compound GSF, since GFF inhibits the adhesion to human melanoma cells more effectively, particularly at concentrations of 0.1 - 5 mM, more particular at concentrations of 0.1, 1, 2.5 and 5 mM. For example, GFF inhibited the adhesion of WM-266-4 cells to fibronectin at a concentration of 5 mM by 62%, while GSF showed an inhibition value of only 26%. Furthermore, GFF showed an inhibition value of 34% already at a concentration of 0.1 mM where GSF showed an inhibition value of only 8% (see Fig. 2). Additionally, GFF also represents a clear improvement with regard to the inhibition of migration of human melanoma cells, particularly at concentrations of 1 - 5 mM, more particular at concentrations of 1 and 2.5 mM. For example, GFF inhibits the migration of the human melanoma cells WM-115 already at a concentration of 1 mM, wherein GSF inhibits the migration of the human melanoma cells only at concentrations greater than or equal to 2.5 mM. Furthermore, GFF shows higher values of inhibition at a concentration of 2.5 mM (see Table 1). Summarizing, GFF shows a better biological activity compared to GSF. In addition, GSSF is much more effective in inhibiting the migration of human melanoma cells than GSF, particularly at concentrations of 0.1 - 5 mM, more particular at concentrations of 0.1, 1, 2.5 and 5 mM. For example, GSSF inhibits the migration of human melanoma cells already at a concentration of 0.1 mM and 1 mM, wherein GSF inhibits the migration of human melanoma cells only at concentrations greater than or equal to 2.5 mM. Furthermore, GSSF shows higher values of inhibition at a concentration of 2.5 mM (see Table 1). Thus, GSSF represents a very useful and promising compound in inhibiting angiogenesis and the formation of metastasis.

Thus, all the compounds GluSF, GFF and GSSF represent an optimization of the known saccharide mimic GSF in inhibiting angiogenesis and the formation of metastasis. The results of the different experiments show that the compounds of the invention inhibit the adhesion of the vascular endothelial cells to the extracellular matrix and/or the migration of the human melanoma cells through the matrix more effectively as observed for GSF. Thus, the compounds of the present invention are optimized and influence the process of angiogenesis already at an early stage and have an enormous potential, since they are not only applicable for inhibiting angiogenesis but also for inhibiting metastasis.

The invention also relates to pharmaceutical compositions containing a therapeutically active amount of one or more (= at least one) of the compounds of the present invention. The compounds of the present invention are therapeutically particularly useful for those diseases where cell migration and neoangiogenesis plays a role. Thus, the treatment of all types of neoplasms and tumors, whether benign or malign, would be possible by the administration of the compounds. Neoplasms may be malignant, e.g. carcinoma, sarcoma, glioblastoma, astrocytoma, neuroblastoma, retinoblastoma, etc. The terms "malignant neoplasm" and "malignant tumor" refer to a neoplasm that contains at least one cancer cell. Neoplastic cells are typically invasive, i.e. they invade adjacent tissues by cell migration or are shed from the primary site and circulate through the blood and lymph to other locations in the body where they initiate one or more secondary cancers (metastases). Thus, the term "cancer" is used herein to refer to a malignant neoplasm, which may or may not be metastatic. In particular, the compounds of the present invention are useful for treating any cancers located in human or animal organs, brain, skin or hematological neoplasia. Examples are melanomas, basal cells carcinoma, Kaposi sarcoma, glioma, cancer of the lung, bladder, prostate, kidney, testis, intestine, colon, spleen, cervix, ovary, endometrium, uterus, esophagus, tongue, bronchi, pancreas, liver, breast, stomach or bone, leukemia (ALL, CLL, AML), lymphoma, myeloma (multiple myelomas, Hodgkin-, non-Hodgkin-). Furthermore, the compounds of the invention are also useful for treating diseases, which are generally involved by neoangiogenesis, such as ophthalmic diseases (macular degeneration, diabetic retinopathy), skin diseases (psoriasis, hemangiomas) and rheumatoid arthritis.

In addition, the compounds of the present invention may be useful as diagnostic tools in any imaging process. For this utility they may carry tags or labels, e.g. fluorescence, radioactivity, ³H, ¹⁴C, ¹⁸F, Iodine. The imaging processes may be computer tomography (CT), magnetic resonance tomography (MRT) or positron emission tomography (PET).

The compounds according to the invention can be administered in various ways, e.g. orally, parenterally, cutaneously, subcutaneously, intravenously, intramuscularly, rectally or by inhalation. The oral in acid resistant formulations (capsules) or inhalational administration is preferred. The compound is given to a patient who is in need of a therapy of a disease coming under the indication spectrum of the compounds according to the invention over a period to be determined by a physician. The compound can be administered to both humans and other mammals.

The dosage of the compounds according to the invention is determined by the physician by means of the patient-specific parameters, such as age, weight, sex, severity of the disease, *etc.* The dosage is preferably between 0.001 mg/kg and 1000 mg/kg body weight, more preferably 0.01 and 500 mg/kg body weight and most preferably 0.1 and 100 mg/kg body weight.

In accordance to the kind of administration, the medicament is formulated suitably, e.g. as solutions or suspensions, simple tablets or coated tablets (dragées), hard or soft gelatine capsules, powder for reconstitution prior to use, aerosols, inhalation sprays, active substance plasters, granules, suppositories, ovules, injectables, creams, ointments, gels, microspheres, implants, which are produced according to conventional galenic processes.

The compounds according to the invention can optionally be formulated with further active substances and with excipients common in pharmaceutical compositions, depending on the preparation to be produced e.g. talcum, gum arabic, lactose, starch, magnesium stearate, cacao butter, aqueous and non-aqueous carriers, adipoids of animal or vegetable origin, paraffin derivatives, glycols (in particular polyethylene glycol), various plasticizers, preservatives, dispersants or emulsifiers.

Additives, such as sodium chloride solution, ethanol, sorbitol, glycerol, olive oil, almond oil, propylene glycol, ethylene glycol or other additives common in pharmacy, can be used for the production of liquid preparations.

When infusion or injectable solutions are used, these are preferably aqueous solutions or suspensions, which can be produced prior to use, e.g. from lyophilized preparations which contain the active substance as such or together with a carrier, such as mannitol, lactose, glucose, albumin and the like. The ready-to-use solutions are sterilized and, if required, mixed with excipients, e.g. preservatives, stabilizers, emulsifiers, solubilizers, buffers and/or salts for regulating the osmotic pressure. The sterilization can be obtained by sterile filtration through filters having a small pore size whereupon the composition may be lyophilized, where necessary. Small amounts of antibiotics can also be added to maintain sterility.

The invention also relates to pharmaceutical compositions containing a therapeutically active amount of the active ingredient together with organic or inorganic solid or liquid pharmaceutically compatible carriers which are suited for the intended administration and which do not unfavourably interact with the active ingredients.

The invention also relates to processes for the production of pharmaceutical compositions, which are characterized in that the compound according to the invention is mixed with a pharmaceutically compatible carrier.

Based on combination therapies with already known active substances, the compounds according to the invention are suited for treating various diseases. Here, unexpected synergistic effects for increasing the therapeutic effectiveness of the substances according to the invention shall be utilized. On the one hand, the combination can consist of offering a single pharmaceutical composition which contains at least one of the compounds according to the invention in combination with one or more of other known active substances or the patient is given several preparations containing one or more of the known active substances simultaneously with, or time-staggered with respect to, the pharmaceutical composition according to the invention. The known active agents may be selected from the group consisting of nucleoside analogs (e.g. fludarabin, cladribin), alkylating agents (e.g. chlorambucil, cyclophosamid), corticosteroids, angiogenesis inhibitors (e.g. bevacizumab, semaxanib, vandetanib) thalidomide, interferon, and anti-cancer antibodies (e.g. Herceptin).

The present invention provides compounds of Formula (I) for example GluSF, D-sulfonic acid, GFF and GSSF, which show a better biological activity as known saccharide mimics in inhibiting angiogenesis and formation of metastasis by inhibiting adhesion and/or migration of human melanoma cells in a concentration dependent manner. Thus, the present invention relates to saccharide mimics, which have been optimized by modifying the structure of the known saccharide mimic GSF and to pharmaceutical compositions comprising these saccharide mimics. Thus, the combination of these routes of optimization allows conclusions about the biological efficiency, biodegradability and toxicity, wherein various novel chemical compounds are provided. In particular, the inhibitory activity with regard to angiogenesis and cell migration which leads to an overall inhibition of metastasis formation could be surprisingly optimized. In this regard the most promising compounds are GSSF and GFF. For both compounds it could be verified in the experiments which are shown in the Examples that the cell migration could be inhibited already at a concentration of 0.1 mM.

The invention is further described with regard to the figures which show:
Fig. 1: GSSF synthesis scheme.
Fig. 2: Inhibition of the adhesion of the human melanoma cells WM-266-4 to fibronectin in the presence of increasing concentrations of GSF, GFF or GluSF
Fig. 3: Inhibition of the adhesion of human endothelial cells HBMEC-60 stimulated with TNF-α to laminin in the presence of increasing concentrations of GSSF in comparison to 10 mM GSF.

The invention is further described with regard to the following examples which are not construed as any limitations of the invention.

### Example 1

### Synthesis of 3-β-D-glucopyranosyl-oxymethyl-4-sulfato-methyl-furan (GluSF)

For the synthesis of GluSF, 1.1 eq. 2,3,4,6-tetra-O-benzoyl-α-D-glucoseimidate was mixed with absolute CH₂Cl₂ and cooled to a temperature of -40°C. Catalytic amounts of Trimethylsilyl trifluoromethanesulfonate (TMS-triflate) were added after the addition of 1 eq. 3,4-bishydroxymethylfuran. Then, the solution was slowly heated for 2 hours to room temperature. Thin layer chromatography (TLC) was performed with a mixture of hexane/ethyl acetate (3:2). The reaction was stopped by adding half-saturated NaHCO₃. The organic phase was washed three times with a solution of saturated NaHCO₃ and three times with water. The solvent was evaporated after drying with Na₂SO₄. The purification was effected by means of column chromatography on silica gel with a mixture of hexane/ethyl acetate (3:2). The monosaccharide 3-(2,3,4,6-tetra-O-benzoyl-β-D-glucopyranosyl-oxymethyl)-4-hydroxymethylfuran was obtained in a yield of 65% as white foam.

In a further reaction step, 1 eq. 3-(2,3,4,6-tetra-O-benzoyl-β-D-glucopyranosyloxymethyl)-4-hydroxymethylfuran was dissolved in just a few millilitres of absolute dimethylformamide (DMF), which were supplemented with 8-10 eq. NMe₃*SO₃, and heated to 55°C for 5 h (TLC-control). The solvent was evaporated and the oily residue was purified by means of the column chromatography on silica gel with a mixture of CH₂Cl₂/CH₃OH (9:1). The product was obtained in a yield of 90% as white foam.

The benzoyl-protected, sulfated saccharide mimic was dissolved in just a few millilitres methanol, supplemented with 0.1 N solution of sodium ethylate and stirred at room-temperature for 10 h. The solvent was evaporated after the reaction has been completed and the residue was adjusted in just a few millilitres of water and almost neutralized to a pH-value of about 7.2 with 0.1 N HC1. The aqueous phase was extracted up to five times with ethyl acetate and finally lyophilized. The purification was effected by means of the column chromatography with a mixture of acetonitrile/H₂O (95:5). The product was obtained in a yield of 88% as white powder.

Mass (ESI): m/z (%) = 369.1 [M-H]-(100).

### Synthesis of 3-β-D-galactopyranosyl-oxymethyl-4-fluoro-methyl-fluoro (GFF)

For the synthesis of GFF, 1.1 eq. 2,3,4,6-tetra-O-benzoyl-α-D-glucoseimidate was mixed with absolute CH₂Cl₂ and cooled to a temperature of -40°C. Catalytic amounts of TMS-triflate were added after the addition of 1 eq. 3,4-bishydroxymethylfuran. Then, the solution was slowly heated for 2 hours to room temperature, wherein the TLC-control was performed with a hexane/ethyl acetate mixture (3:2). The reaction was stopped by adding half-saturated NaHCO₃. The organic phase was washed three times with a solution of saturated NaHCO₃ and three times with water. The solvent was evaporated after drying with Na₂SO₄. The purification was effected by means of column chromatography on silica gel with a mixture of hexane/ethyl acetate (3:2). The monosaccharide 3-(2,3,4,6-tetra-O-benzoyl-β-D-glucopyranosyl-oxymethyl)-4-hydroxymethylfuran was obtained in a yield of 70% as a white foam.

In a further reaction step, 1 eq. 3-(2,3,4,6-tetra-O-benzoyl-β-D-glucopyranosyloxymethyl)-4-hydroxymethylfuran was dissolved in just a few millilitres of absolute DMF, cooled to 0°C by means of an ice bath and supplemented with 2 eq. DAST by means of a septum. The reaction was stopped after 2 h by adding of just a few millilitres of methanol. The purification was effected by means of a column chromatography with a mixture of hexane/ethyl acetate (3:2). The product was obtained in a yield of 80% as white foam.

The benzoyl-protected, halogenated saccharide mimic 3-(2,3,4,6-tetra-O-benzoyl-β-D-galactopyranosyl-oxymethyl)-4-fluoro-methyl-furan was dissolved in just a few millilitres methanol, supplemented with a freshly prepared solution of 0.1 N sodium ethylate and stirred at room-temperature for 10 h (TLC-control). The solvent was evaporated after the reaction has been completed and the residue was adjusted in just a few millilitres of water and neutralized to a pH-value of about 7.0 with 0.1 N HCl. The aqueous phase was extracted up to five times with ethyl acetate and finally lyophilized. The purification was effected by means of the column chromatography with a mixture of acetonitrile /H₂O (90:10). The product was obtained in a yield of 83% as white powder.

Mass (ESI): m/z (%) = 315.1 [M+Na]⁺ (100), 607.2 [2M+Na]⁺ (21).

### Synthesis of 3-(6-sulfato-β-D-galactopyranosyl-oxymethyl)-4-(sulfatometyhl)-furan (GSSF)

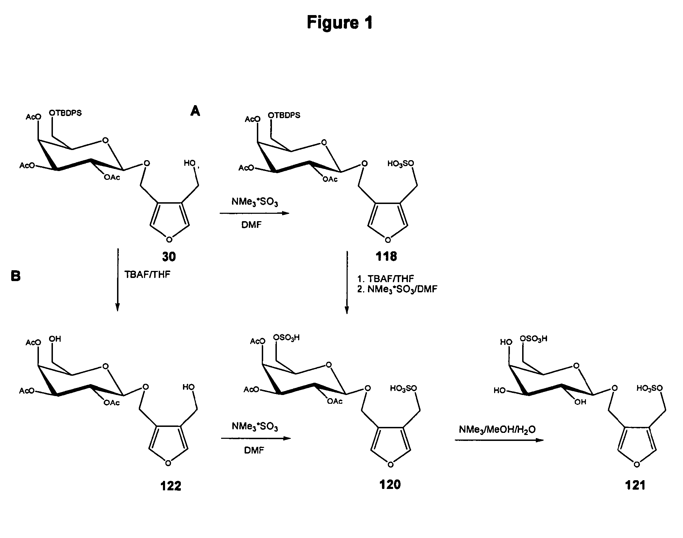

For the synthesis of GSSF (see Fig. 1), 1-Trichloroacetimido-2,3,4-tri-O-acetyl-6-O-tert-butyldiphenylsilyl-galactopyranoside was reacted with 3,4-bishydroxymethylfuran and trimethylsilyl trifluoromethanesulfonate (TMSOTf) at a temperature of about 0°C in absolute CH₂Cl₂. The products monogalactoside and bigalactoside were obtained as white needles in a yield of about 71 % and 6 %, respectively. One eq. monoglycosidized furan was reacted with 5 eq. NMe₃ *SO₃ in DMF at a temperature of about 55°C, wherein the sulfation has been completed after 5 h incubation. The purification was effected by means of column chromatography on silica gel with a mixture of CH₂Cl₂/MeOH (5:1). The product **118** is obtained in a yield of about 95%. For deprotection (cleavage of the tert-butyl diphenylsilyl-protecting group), the product was reacted with tetra-n-butylammonium fluoride in tetrahydrofuran at 0°C. The purification was effected by means of a column chromatography on silica gel with a mixture of CH₂Ch/MeOH (5:1), wherein the product was obtained in a yield of about 51%. The sulfation was effected with 5 eq. NMe₃ *SO₃ in DMF at a temperature of about 55°C. The purification of the double sulfated, acetyl-protected raw product was effected by means of a column chromatography on silica gel with a mixture of CH₂Cl₂/MeOH (95:5). The intermediate product 120 was obtained, as a clear oil, in a yield of about 80%. For deprotection (cleavage of the acetyl-protecting groups), the product was reacted with a mixture of NMe₃/MeOH/H₂O (4:3:1). The solvent was evaporated after 12 h reaction time and was further evaporated with ethanol. The purification was effected by means of column chromatography on silica gel with a mixture of acetonitrile/H₂O (95:5). The saccharide mimic GSSF 121 was obtained in a yield of 65% as white powder.

### Example 2

### Adhesion Assay with WM-266-4 cells

Microtiter plates with 96 wells were coated with 0.5 µg of fibronectin in 50 µl over night at 4° C and blocked with 200 µl adhesion buffer (1% BSA in PBS without Ca²⁺/Mg²⁺). WM-266-4 cells were pre-incubated with the test compounds at the respective concentrations in serum free medium for 60 min in a shaker (600 rpm) at 37° C. Afterwards, 4 x 10⁴ cells in 100 µl were withdrawn and added into each well and incubated for 1 h at 37° C. Medium and non-adherent cells were aspirated and adherent cells were fixed in 4% formalin in PBS. The plates were washed with 0.01 M borate buffer (Stock-solution: 20 mM boric acid, 100 mM sodium tetraborate; pH 8.5; working solution: 0.01 M dilution in H₂O) and 50 µl of 0.01 % methylene blue in borate buffer were added, wherein the cells were stained for 10 min at room temperature. The plates were extensively washed with water and air-dried. The air-dried plates were incubated for 45 min with 200 µl/well extraction buffer (a mixture of 0.1 N HCl and absolute EtOH (1:1, vol/vol)), wherein the adherent cells were quantified in an ELISA-reader, which read the extinction at 651 nm.

The human melanoma cells adhered to the fibronectin coated cell culture dishes. This adhesion is a prerequisite for growth of circulating cells such as occurs when these metastasize to sites distant from the tumor. The compounds described herein can inhibit such adhesion to the extracellular matrix protein fibronection or laminin to various degrees. Figure 2 shows the results for the inhibition of the adhesion of WM-266-4 cells using GluSF, GFF, and GSSF compared to GSF, respectively.

With regard to the adhesion of human melanoma cells to ECM, GluSF was at least as effective as GSF, since GluSF inhibited the adhesion of human melanoma cells to ECM at least as strong as GSF, particularly at concentrations of 0.1 - 5 mM, more particular at concentrations of 0.1, 1, 2.5 and 5 mM. For example, at a concentration of 0.1 mM, GSF and GluSF were equally effective, wherein GluSF was much more effective at concentrations between 1 and 5 mM, more particular at concentrations of 1, 2.5 and 5 mM. At a concentration of 5 mM, GluSF inhibited the adhesion of WM-266-4 cells to fibronectin by 79%, while GSF showed an inhibition value of only 26% (see Fig. 2).

Furthermore, GFF inhibited the adhesion to human melanoma cells more effectively than GSF, particularly at concentrations of 0.1 - 5 mM, more particular at concentrations of 0.1, 1, 2.5 and 5 mM. For example, GFF inhibited the adhesion of WM-266-4 cells to fibronectin at a concentration of 5 mM by 62%, while GSF showed an inhibition value of only 26%. Furthermore, GFF showed an inhibition value of 34% already at a concentration of 0.1 mM where GSF showed an inhibition value of only 8% (see Fig. 2).

### Adhesion Assay with HBMEC cells

Microtiter plates with 96 wells coated with the extracellular matrix component type I collagen, fibronenctin or laminin were used. HBMEC-60 cells which had been pre-incubated with the tumor necrosis factor (TNF-α, 40 ng/ml) for 24 hrs were incubated with the test compounds at the respective concentrations in serum free medium for endothelial cells (Promozell, Heidelberg, Germany) without growth factors for 30 min in 1.5 mL microfuge tubes (Eppendorf, Hamburg, Germany). Afterwards, 4 x 10⁴ HBMEC-60 cells in 100 µl were withdrawn and added into each well. The plates were incubated at 37° C for 30 min and subsequently washed twice with PBS (with CaCl₂ und MgSO₄). Then, the adherent cells were fixed, washed and stained with methylene blue, the dye released and the extinction read as described above for the experiments with the melanoma cells.

Adhesion of endothelial cells to extracellular matrix proteins like laminin is a prerequisite for angiogenesis. An inhibition by a non-cytotoxic compound like GSSF would effectively inhibit angiogenesis and thereby tumour growth.

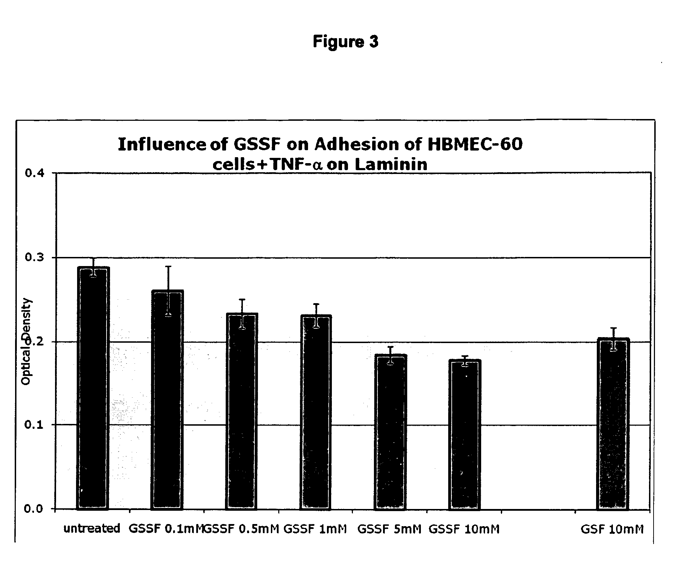

Reference is made to Figure 3 which shows that 10 mM GSSF was a better inhibitor of HBMEC-60 cell adhesion to laminin than 10 mM GSF. But also at concentrations of 0.1, 0.5, 1 and 5 mM GSSF significantly inhibited endothelial cell adhesion to laminin. GSSF is therefore a better inhibitor than GSF.

### Example 3

### Wound Healing Assay

The wound healing assay has been carried out according to description as disclosed in Yarrow, J.C., et al., BMC Biotechnol., 2004: 4, 21-29. For carrying out the wound healing assay, 2 x 10⁵ cells were grown in one well of a 12-well-plate under cell culture specific conditions for 24 h. After 24 h, a wound has been scratched in the confluently grown cell monolayer with a 100 µl pipette-tip. Detached cells were removed by washing with serum-free medium and new media with the respective compounds to be tested were supplemented with varying amounts. The cells were incubated under cell culture specific conditions and always the same section was photographed after 2, 4, 8 and 24 under the phase contrast microscope. The untreated cells healed the wound through a combination of proliferation and migration after 24 h.

The following table (Table 1) is a summary of the results obtained by carrying out the above described wound healing assay with varying amounts of compounds of the present invention to inhibit the cell migration of WM-115 cells, wherein (-) means that the cell migration was not inhibited and that the wound healed, (+) means a slight inhibition, which slows the trend to heal, (++) means that the cell migration was inhibited, since only some cells were visible in the wound, and (+++) means the absolute inhibition of cell migration. The abbreviation "n.d." means "not determined".

**Table 1.**

| **Structure** | **Concentration** | | | |
|---|---|---|---|---|
| | **0.1 mM** | **1 mM** | **2.5 mM** | **5mM** |
| **GSF** | - | - | + | +++ |
| **GluSF** | - | + | + | +++ |
| **GFF** | n.d. | + | +++ | +++ |
| **GSSF** | ++ | ++ | +++ | +++ |

It could be observed that GluSF was more effective in inhibiting the migration of human melanoma cells compared to GSF, particularly at concentrations of 1 - 5 mM, more particular at concentrations of 1, 2.5 and 5 mM. For example, GluSF inhibited the migration of human melanoma cells already at a concentration of 1 mM, wherein GSF inhibited the migration of human melanoma cells only at concentrations greater than or equal to 2.5 mM (see Table 1). Such inhibition does not represent a cytotoxic effect of the saccharide mimic, since the vitality test with Trypanblue showed that the cells were intact.

Additionally, GFF showed an improvement with regard to the inhibition of migration of human melanoma cells, particularly at concentrations of 1 and 2.5 mM. For example, GFF inhibited the migration of the human melanoma cells WM-115 already at a concentration of 1 mM, wherein GSF inhibited the migration of the human melanoma cells only at concentrations greater than or equal to 2.5 mM. Furthermore, GFF showed much higher values of inhibition at a concentration of 2.5 mM (see Table 1). Such inhibition does not represent a cytotoxic effect of the saccharide mimic, since the vitality test with Trypanblue showed that the cells were intact.

Another optimization of the saccharide mimic GSF leads to the compound GSSF, which contains 3,4-bishydroxymethylfuran as the core-molecule and a sulfate-group at the core-molecule and at position 6 of the saccharide galactose, respectively. GSSF was much more effective in inhibiting the migration of human melanoma cells than GSF, particularly at concentrations of 0.1 - 5 mM, more particular at concentrations of 0.1, 1, 2.5 and 5 mM. For example, GSSF inhibited the migration of human melanoma cells already at a concentration of 0.1 mM, wherein GSF inhibited the migration of human melanoma cells only at concentrations greater than or equal to 2.5 mM (see Table 1). The vitality test with Trypanblue showed that the cells were intact.

## Claims

1. A compound of the general formula (I) 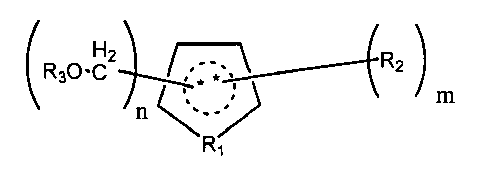
n, m = 1,2,3 with n + m≤4
R₁ = N-H or O
R₂ = Alkyl-R₄
R₃ = saccharide selected from the group consisting of glucose, galactose, and the corresponding uronic acids, optionally having at least one of the saccharide-OH groups substituted by a R₄ residue and/or having at least one of the saccharide-OH groups oxidized to O
R₄ = H, halogen, -NH-C(O)-R₅, -N(R₆)₂, NR₆, NO₂, -O-S(= O)₂-O-R₆, S(= O)₂-O-R₆, -O-P(= O)(-O-R₆)₂, -OR₆, -O-C(O)-R₅, -S-R₆
R₅ = linear C₁ to C₄ alkyl
R₆ = H, linear C₁ to C₄ alkyl
with the exception of a compound where R₁ = O, R₂ = CH₂-O-S (= O)₂ -O-H, R₃ = unsubstituted galactose, m = 1, n = 1.

2. The compound of claim 1, wherein the compound is 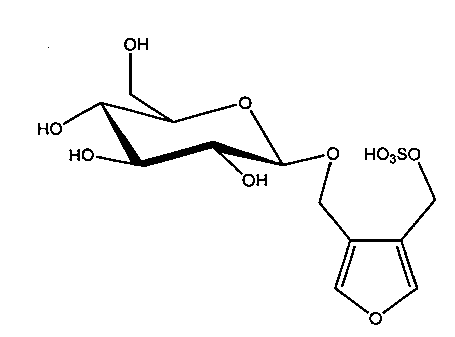 or 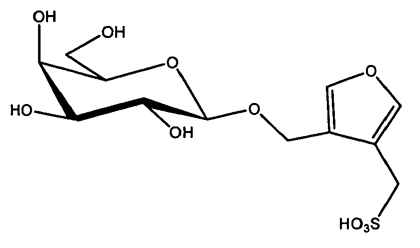 or 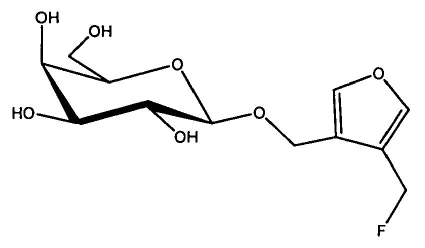 or

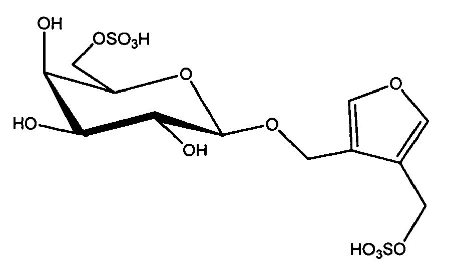

3. A pharmaceutical composition comprising at least one compound of claim 1 or 2 together with pharmaceutically acceptable carriers and/or excipients.

4. The pharmaceutical composition of claim 3 comprising further active agents selected from the group consisting of nucleoside analogs, alkylating agents, corticosteroids, angiogenesis inhibitors, interferon and anti-cancer antibodies.

5. Use of a compound of the general formula (I) 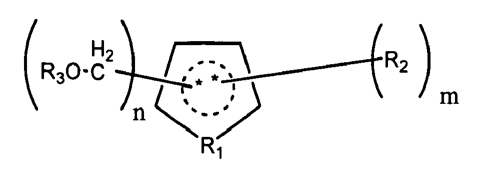
n, m = 1, 2, 3 with n + m ≤ 4
R₁ = N-H or O
R₂ = Alkyl-R₄
R₃ = saccharide selected from the group consisting of glucose, galactose, and the corresponding uronic acids, optionally having at least one of the saccharide-OH groups substituted by a R₄ residue and/or having at least one of the saccharide-OH groups oxidized to O
R₄ = H, halogen, -NH-C(O)-R₅, -N(R₆)₂, NR₆, NO₂, -O-S(= O)₂-O-R_{b}, S(=O)₂-O-R₆, -O-P(= O)(-O-R₆)₂, -OR₆, -O-C(O)-R₅, -S-R₆
R₅ = linear C₁ to C₄ alkyl
R₆ = H, linear C₁ to C₄ alkyl
as a pharmaceutical agent for use in a method of inhibiting cell migration and angiogenesis to avoid tumor growth and the formation of cancer metastases.

6. The use of claim 5, wherein the cancer is melanoma, basal cells carcinoma, Kaposi sarcoma, glioblastoma, neuroblastoma, cancer of the lung, bladder, prostate, kidney, testis, intestine, colon, spleen, cervix, ovary, endometrium, uterus, esophagus, tongue, bronchi, pancreas, liver, breast, stomach or bone, leukemia, lymphoma or myeloma.

7. A pharmaceutical composition comprising at least one compound of claim 5 or 6 together with pharmaceutically acceptable carriers and/or excipients.

8. The pharmaceutical composition of claim 7 comprising further active agents selected from the group consisting of nucleoside analogs, alkylating agents, corticosteroids, angiogenesis inhibitors, interferon and anti-cancer antibodies.

9. Use of a compound of the general formula (I) 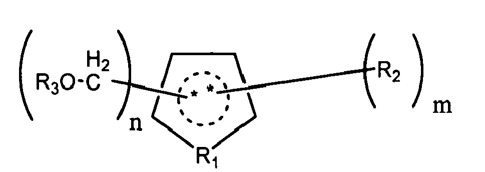
n, m = 1,2,3 with n + m ≤ 4
R₁ = N-H or O
R₂ = Alkyl-R₄
R₃ = saccharide selected from the group consisting of glucose, galactose, and the corresponding uronic acids, optionally having at least one of the saccharide-OH groups substituted by a R₄ residue and/or having at least one of the saccharide-OH groups oxidized to O
R₄ = H, halogen, -NH-C(O)-R₅, -N(R₆)₂, NR₆, NO₂, -O-S(= O)₂-O-R₆, S(=O)₂-O-R₆, -O-P(= O)(-O-R₆)₂, -OR₆, -O-C(O)-R₅, -S-R₆
R₅ = linear C₁ to C₄ alkyl
R₆ = H, linear C₁ to C₄ alkyl
for diagnosing cancer in an imaging process.
